# EUROPEAN PATENT SPECIFICATION

(11) **EP 3 896 493 B1**
(45) Date of publication and mention of the grant of the patent: **04.11.2026**
(21) Application number: 19900092.8
(22) Date of filing: 28.11.2019
(51) Int. Cl.: G01T 1/20

(54) **GAMMA RADIATION IMAGING DEVICE AND IMAGING METHOD**
VORRICHTUNG UND VERFAHREN ZUR BILDERZEUGUNG MITTELS GAMMASTRAHLUNG
DISPOSITIF D'IMAGERIE PAR RAYONNEMENT GAMMA ET PROCÉDÉ D'IMAGERIE

(30) Priority: 19.12.2018 CN 201811559943
(43) Date of publication of application: 20.10.2021
(73) Proprietor: Tsinghua University, Beijing 100084 (CN)
(72) Inventor: MA, Tianyu, Beijing 100084 (CN); LIU, Yaqiang, Beijing 100084 (CN); WANG, Xuewu, Beijing 100084 (CN); WANG, Zhong, Beijing 100084 (CN)
(74) Representative: Gulde & Partner
(86) International application number: PCT/CN2019/121615
(87) International publication number: WO 2020/125371

(56) References cited:
- WO-A1-2015/044019
- CN-A- 103 558 626
- CN-A- 108 139 491
- CN-A- 108 139 491
- DE-A1- 10 318 416
- JP-A- 2009 063 589
- JP-A- 2013 200 164
- US-A- 4 037 105
- US-A1- 2010 301 221
- US-A1- 2018 172 848

## Description

### TECHNICAL FIELD

The present invention relates to the field of nuclear technology and application technology, and in particular to a gamma radiation imaging device and imaging method thereof.

### BACKGROUND

Gamma radiation imaging is widely used in medical diagnosis, nuclear leakage and nuclear radiation hot spot monitoring, nuclear waste management, industrial and agricultural radioactive source management and monitoring. The gamma radiation imaging device is used to detect the nuclide that emits gamma photons and form an image of its spatial distribution. It may be used independently as an industrial gamma camera, or as a gamma camera for medical diagnosis, or as key functional components of single-photon emission computed tomography (SPECT) or positron emission computed tomography (PET).

Gamma radiation imaging devices generally include a detector and a collimator. Where the detector part adopts a position-sensitive gamma detector to obtain the position information, energy information, and time information of the photons incident on the detector, which may be a scintillation detector composed of a scintillation crystal + photomultiplier tube, or a semiconductor detector, or other detectors that may be used for gamma radiation measurement. The collimator is disposed between the detector and the object to be detected. It only allows photons in a certain direction to be incident on the detector and absorb photons in other directions. Combining the position of the photons detected on the detector and the incident direction of the photons allowed by the collimator, the path information of the photons emitted from the human body may be obtained to form a plane gamma radiation source distribution image. It is also possible to use the detector and collimator to rotate around the object to be imaged, measure multiple plane gamma images in multiple directions, and obtain three-dimensional gamma radiation source distribution images by using tomographic reconstruction algorithms.

The collimator of the gamma radiation imaging device adopts the principle of absorption collimation. That is, a collimator is made of heavy metals such as lead and tungsten. The collimator is provided with holes, slits, grooves, and other gaps. The photons that enter the gaps pass through the collimator and are detected by the detector. Other photons are blocked and absorbed by the collimator. Typically, there are parallel-hole collimators, fan-beam collimators, pinhole collimators, etc. The collimator made in this way blocks most of the photons and allows only a small part of the photons to be passed through, so that the photon events received on the detector unit may only come from a smaller area in the space of the object to be imaged, and images with higher spatial resolution may be obtained through image reconstruction algorithms. However, as a large number of photons are absorbed, the detection efficiency is very low, which seriously affects the imaging performance.

The gamma radiation imaging device based on the coded aperture collimator greatly improves the aperture ratio on the collimator. A large number of photons incident from radiation sources in different directions form different projection plane distributions on the detector and the image reconstruction algorithm is used to solve the direction of the radiation sources. Although the detection efficiency of this kind of collimator is greatly improved, the photon events received on the detector unit may come from multiple areas or a larger area in the space of the object to be imaged, and the direction information that may be obtained from a single photon is significantly reduced, only suitable for imaging of specific distributions such as spot or sparse radioactive sources. In scenes such as nuclear medicine imaging, due to the wide and continuous distribution of radiopharmaceuticals in the human body, the imaging effect is worse than that of gamma cameras with low detection efficiency based on parallel-hole collimators and other collimators.

In summary, as the collimator of the traditional gamma radiation imaging device adopting the absorption collimation principle absorbs a large number of photons, the detection efficiency of the imaging device is very low, which makes the acquisition time long, or the image quality in the limited acquisition time is poor. The coded aperture collimator with high aperture ratio improves the detection efficiency, but reduces the directional information carried by the received photon events, and its image quality has not been improved accordingly.

US 4 037 105 A relates to an electromagnetic radiation detector which can detect the location as well as measure the energy of a source of radiation; without the use of a collimator and with the use of only a single photomultiplier tube. WO 2015/044019 A1 discloses a multimodal imaging apparatus for imaging a radiotracer distribution within a subject. US 2010/301221 A1 provides a gamma ray detector and a gamma ray reconstruction method which can be used in SPECT and PET and which combine and reconstruct the information on "Compton-scattered" gamma rays. JP 2013 200164 A discloses a gamma ray detector which detects the gamma ray released from the single photon radioisotope, and a diagnostic apparatus provided with the same. JP 2009 063589 A provides a radiation source position detection system and a radiation source position detecting probe for detecting the existing position of a radiation source which are used in a nuclear power development field, a medical field, and the like.

### SUMMARY

### (1) Technical problems to be solved

In view of the above problems, the main purpose of the present invention is to provide a gamma radiation imaging device and imaging method that not only has high photon detection efficiency, but also high photon events information, so as to solve at least one of the above problems.

### (2) Technical solution

In order to achieve the above purpose, the present invention provides a gamma radiation imaging device with separate detectors according to claim 1 and an imaging method according to claim 7. By separating the detectors into multiple units in space, and placing different detector units in sequence along the movement direction of the photons, a detector unit located in front along the movement direction of the photons may block and collimate photons for a detector unit following the detector unit.

The photon events measured by all the detector units in the above device (including the detector units that have collimating effect on other detectors) may be applied to any imaging method, thereby improving the detection efficiency and increasing the directional information carried by the photon events, resulting in higher quality images.

According to one aspect of the present disclosure which does not form part of the appended claims, there is provided a gamma ray imaging device, including: a plurality of separate detectors, wherein rays emitted from different positions in an imaging area reach at least one of the plurality of separate detectors through different sets of one or more other detector of the plurality of separate detectors, the sets of one or more other detector are different in at least one of thickness of detector and number of detectors.

According to another aspect of the present disclosure which does not form part of the appended claims, there is provided an imaging device, including: a plurality of separate detectors, the plurality of separate detectors form a plurality of detector layers arranged outside an object to be detected, wherein two adjacent detector layers are spaced from each other by an interval.

According to yet another aspect of the present disclosure which does not form part of the appended claims, there is provided an imaging device including: a plurality of separate detectors, the plurality of separate detectors comprise at least two types of detectors, the plurality of separate detectors form a plurality of detector layers arranged outside an object to be detected, and the imaging device further comprises a collimator located between the object to be detected and an innermost detector layer along movement direction of photons.

According to another aspect of the present disclosure which does not form part of the appended claims, there is provided an imaging method, including: providing a plurality of separate detectors, arranging the plurality of separate detectors in form of multi-layers outside an object to be detected to form a plurality of detector layers; imaging the object to be detected by using the plurality of detector layers; wherein rays emitted from different positions in an imaging area reach at least one of the plurality of separate detectors through different sets of one or more other detector of the plurality of separate detectors, the sets of one or more other detector are different in at least one of thickness of detector and number of detectors.

According to another aspect of the present disclosure which does not form part of the appended claims, there is provided an imaging method, including: providing a plurality of separate detectors, arranging the plurality of separate detectors in form of multi-layers outside an object to be detected to form a plurality of detector layers, wherein two adjacent detector layers are spaced from each other by an interval; imaging the object to be detected by using the plurality of detector layers.

According to another aspect of the present disclosure which does not form part of the appended claims, there is provided an imaging method, including: providing a collimator and at least two types of a plurality of separate detectors; arranging the plurality of separate detectors in form of multi-layers outside an object to be detected to form a plurality of detector layers; disposing the collimator between the object to be detected and a detector layer located innermost along movement direction of photons; imaging the object to be detected by using the plurality of detector layers and the collimator.

### (3) Effects

It may be seen from the above technical solutions that the gamma radiation imaging device with separate detectors and imaging method of the present invention have at least one of the following beneficial effects.
(1) The detector unit used to detect photons may be used as a collimator of other detector units at the same time, so that rays from different directions incident on a certain detector unit may have different attenuation ratios, because they pass through different numbers of other detectors or different thicknesses of other detector units, which may reduce the absorption loss of photons on the collimator and improve the effect of determining the direction of the photons and the imaging quality. Therefore, it has both high photon detection efficiency and high photon events information.
(2) The detectors may be divided into multiple layers in space, and by changing the spatial arrangement of the detector units and/or changing the intervals between the detector units, the rays from different directions incident on a certain detector unit may have different attenuation ratios, because they pass through different other detector units on their paths, thereby reducing the absorption loss of photons on the collimator and improving the effect of determining the direction of the photons and the imaging quality.
(3) According to an aspect of the present disclosure which does not form part of the appended claims, the detectors may be composed of a variety of materials, and the different photon attenuation ratios of different materials may improve the effect of determining the direction of the photons and the imaging quality, so that the rays from different directions incident on a certain detector unit may have different attenuation ratios, because they pass through different materials of detectors on their paths, thereby reducing the absorption loss of photons on the collimator and improving the effect of determining the direction of the photons and the imaging quality.

### BRIEF DESCRIPTION OF THE DRAWINGS

FIG. 1 is a schematic structural diagram of a gamma radiation imaging device according to an embodiment of the disclosure which does not form part of the appended claims.
FIG. 2 is a schematic structural diagram of a gamma radiation imaging device according to another embodiment of the present invention.
FIG. 3 is a schematic structural diagram of a gamma radiation imaging device according to another embodiment of the disclosure which does not form part of the appended claims.
FIG. 4 is a schematic structural diagram of a gamma radiation imaging device according to another embodiment of the disclosure which does not form part of the appended claims.
FIG. 5 is a schematic structural diagram of a gamma radiation imaging device according to another embodiment of the disclosure which does not form part of the appended claims.
FIG. 6 is a schematic structural diagram of a gamma radiation imaging device according to another embodiment of the disclosure which does not form part of the appended claims.

### <Symbol Description>

1-the first type of detector, 2-the second type of detector, 3,4-light, 5,6-position, 7-collimator, O-object to be detected.

### DETAILED DESCRIPTION OF EMBODIMENTS

In order to make the objectives, technical solutions, and advantages of the present invention clearer, the present invention will be further described in detail below in conjunction with specific embodiments and with reference to the accompanying drawings.

The present invention proposes to use a detector capable of detecting scintillation photons to form a collimator of a gamma radiation imaging device. The collimator part may be composed entirely of detectors that may detect scintillation photons, or may be composed of any existing collimator and a detector that may detect scintillation photons. Any detector in the detector part may be used as a collimator of other detectors, or may only operate as a detector. Through proper detector structure, material, arrangement relationship, etc., at least one of the thicknesses of the detectors and the numbers of detectors that the rays emitted from different positions in the imaging area reach the same detector are different (for example, for any detector a, before the ray b and ray c emitted from different positions in the imaging area reaching the detector a, the thicknesses of the detector passed through are different, and/or the numbers of detectors passed through are different), so that the position of the ray emitted may be determined by measuring the intensity of the ray in the detector, and an image may be obtained.

In order to further enhance the collimator effect of the detector used as a collimator, in an embodiment which does not form part of the appended claims as such, the detector may be composed of a variety of detector materials, so that the detectors at different positions in space may have different photon (ray) attenuation ratios (attenuation coefficients). The rays from different directions incident on a certain detector have different attenuation ratios because they pass through different detector materials on their paths, and thus the purpose of determining the direction of photons may be achieved.

In order to further enhance the collimator effect of the detector used as a collimator, the detectors may be arranged non-closely in space. By changing the distance between the detectors, the distance between adjacent layers is equal to or greater than the size of the detector, or the distance between adjacent detectors on the same layer is equal to or greater than the size of the detector, so that the rays from different directions incident on a certain detector have different attenuation ratios because they pass through different other detectors on their paths, and thus the purpose of determining the direction of photons may be achieved.

### Embodiment 1 (not forming part of the appended claims)

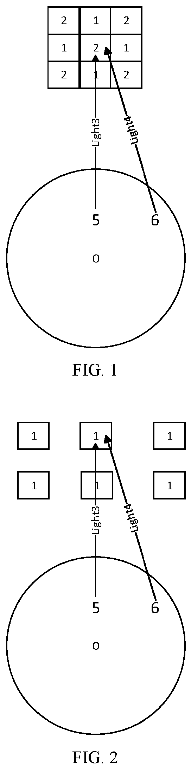

In this embodiment, as shown in FIG. 1, the imaging device includes 9 detectors, and the 9 detectors form 3 detector layers, which are distributed in 3 layers outside the object to be detected (such as a human body). From inside to outside are a first detector layer, a second detector layer, and a third detector layer. The 9 detectors include a total of two types of detectors, a first type of detector 1 and a second type of detector 2. Moreover, any two adjacent detectors have different attenuation ratios to the photons.

Specifically, the first detector and the second detector are made of different materials. With the imaging device of this embodiment, a light 3 from a position 5 of the object to be detected O and a light 4 from a position 6 of the object to be detected O respectively pass through the first type of detector 1 and the second type of detector 2 in the first detector layer before being incident on the second type of detector 2 in the second detector layer, which causes different attenuations, so that probabilities of the photons incident on the second type of detector 2 in the second detector layer come from the position 5 and the position 6 are different, thereby playing the role of determining the direction of the photon. The position 5 and the position 6 are two different positions inside the object to be detected O.

It should be noted that, the number of detectors, the number of detector layers, and the number of detector types in this embodiment are merely exemplary descriptions, that is, the number of detectors is not limited to 9, and the number of detector layers is also not limited to three layers, and the types of detectors are not limited to two types, and those skilled in the art may adjust them appropriately as needed.

### Embodiment 2

In this embodiment, as shown in FIG. 2, the imaging device includes 6 detectors, and the 6 detectors form 2 detector layers, which are distributed in 2 layers outside the object to be detected (such as a human body). From inside to outside are a first detector layer and a second detector layer. The six detectors are all the first type of detector 1. Moreover, there is an interval between any two adjacent detectors, that is, there is an interval between the first detector layer the second detector layer, there is an interval between two adjacent detectors of the first detector layer, and there is also an interval between two adjacent detectors of the second detector layer.

Specifically, with the imaging device of this embodiment, the light 3 from the position 5 of the object to be detected and the light 4 from the position 6 of the object to be detected before being incident on the detector in the second detector layer, the light 3 from the position 5 of the object to be detected passes through the detector of the first detector layer, and the light 4 from the position 6 of the object to be detected only passes through the air, which causes different attenuations, so that probabilities of the photons incident on the detector in the second detector layer come from the position 5 and the position 6 are different, thereby playing the role of determining the direction of the photon.

It should be noted that, the number of detectors, the number of detector layers, and the number of detector types in this embodiment are merely exemplary descriptions, that is, the number of detectors is not limited to 6, and the number of detector layers is also not limited to two layers, and the type of the detector is not limited to type 1, and those skilled in the art may adjust them appropriately as needed.

### Embodiment 3 (not forming part of the appended claims)

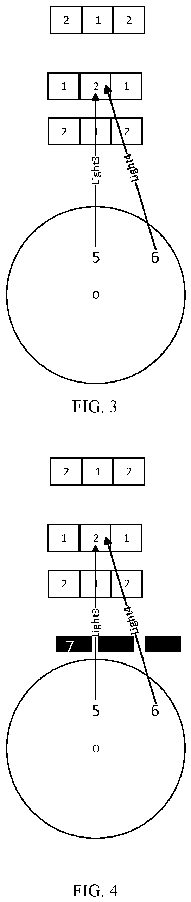

In this embodiment, as shown in FIG. 3, the imaging device includes 9 detectors, and the 9 detectors form 3 detector layers, which are distributed in 3 layers outside the object to be detected (such as a human body). From inside to outside are a first detector layer, a second detector layer, and a third detector layer. The 9 detectors include a total of two types of detectors, a first type of detector 1 and a second type of detector 2. There is an interval between two adjacent detector layers, and an interval between the first detector layer and the second detector layer may be different from an interval between the second detector layer and the third detector layer. Any two adjacent detectors have different attenuation ratios to the photons. The layers are separated by a certain distance to further improve the effect of determining the direction of photons.

Specifically, with the imaging device of this embodiment, the light 3 from the position 5 of the object to be detected and the light 4 from the position 6 of the object to be detected before being incident on the second type of detector 2 in the second detector layer, the light 3 from the position 5 of the object to be detected passes through the first type of detector 1 in the first detector layer, and the light 4 from the position 6 of the object to be detected passes through the second type of detector 2 in the first detector layer, which causes different attenuations, so that probabilities of the photons incident on the second type of detector 2 in the second detector layer come from the position 5 and the position 6 are different, thereby playing the role of determining the direction of the photon.

It should be noted that, the number of detectors, the number of detector layers, and the number of detector types in this embodiment are merely exemplary descriptions, that is, the number of detectors is not limited to 9, and the number of detector layers is also not limited to three layers, and the types of detectors are not limited to two types, and those skilled in the art may adjust them appropriately as needed.

### Embodiment 4 (not forming part of the appended claims)

In this embodiment, as shown in FIG. 4, the gamma radiation imaging device of the composite detector/collimator includes 9 detectors, and the 9 detectors form 3 detector layers, which are distributed in 3 layers outside the object to be detected (such as a human body). From inside to outside are a first detector layer, a second detector layer, and a third detector layer. The 9 detectors include a total of two types of detectors, the first type of detector 1 and the second type of detector 2. There is an interval between two adjacent detector layers, and an interval between the first detector layer and the second detector layer may be different from an interval between the second detector layer and the third detector layer. Any two adjacent detectors have different attenuation ratios to the photons. The layers are separated by a certain distance to further improve the effect of determining the direction of photons.

Further, the imaging device further includes an absorption collimator 7 with a high aperture ratio between the first detector layer and the object to be detected. In the imaging device of this embodiment, except for the first layer of detectors, the other layers of detectors have the effect of determining the direction of the photons because they are attenuated by the previous layers of detectors. By providing an absorption collimator with a high aperture ratio between the first detector layer and the object to be detected, the effect of determining the direction of the photons by the detectors in the first detector layer is further improved.

It should be noted that, the number of detectors, the number of detector layers, and the number of detector types in this embodiment are merely exemplary descriptions, that is, the number of detectors is not limited to 9, and the number of detector layers is also not limited to three layers, and the types of detectors are not limited to two types, and those skilled in the art may adjust them appropriately as needed.

In addition, this embodiment includes an imaging device with a collimator, and the type and arrangement of the detectors may be the same as those in the previous embodiment, and will not be repeated here.

### Embodiment 5 (not forming part of the appended claims)

In this embodiment, the gamma radiation imaging device includes a detector unit, and the detector unit includes four detector array layers, which are a first detector array layer, a second detector array layer, a third detector array layer, and the fourth detector array layer. Each detector array layer includes two types of detectors, which are a first detector and a second detector. The first detector includes NaI inorganic scintillator, and the second detector includes LSO inorganic scintillator. In each detector array layer, the first detector and the second detector are arranged alternately. Each first detector in the first detector array layer is opposite to the position of each second detector in the second detector array layer, and each second detector in the first detector array layer is opposite to the position of each first detector in the second detector array layer. The photons emitted from different positions in the object to be detected may pass through the two materials with different attenuation ratios in the detector unit to form different distributions.

### Embodiment 6 (not forming part of the appended claims)

In this embodiment, the gamma radiation imaging device includes a detector unit, and the detector unit includes four detector array layers, which are a first detector array layer, a second detector array layer, a third detector array layer, and the fourth detector array layer. Each detector array layer includes two types of detectors, which are a first detector and a second detector. The first detector is a GSO detector, and the second detector is an YSO detector. The photons emitted from different positions in the object to be detected may pass through the two materials with different attenuation ratios in the detector to form different distributions.

### Embodiment 7 (not forming part of the appended claims)

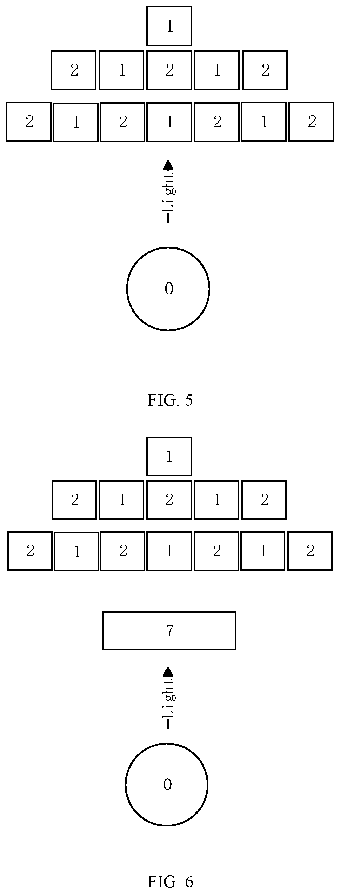

In this embodiment, the gamma radiation imaging device includes multiple detector array layers, and each detector array layer includes multiple types of detectors. Where the maximum distance from the object to be detected, that is, the outermost detector array layer includes one type of detector. The detector array layers in the multiple detector array layers except the outermost detector array layer includes multiple types of detectors. The multiple types of detectors in the same detector array layer are arranged alternately, as shown in FIG. 5.

### Embodiment 8 (not forming part of the appended claims)

The difference from embodiment 7 is that the imaging device of this embodiment further includes a collimator between the first detector layer and the object to be detected, as shown in FIG. 6.

The gamma radiation imaging device adopting the separate detector of the present disclosure has better collimation effect, a greater number of gamma photons are measured, and the spatial resolution and detection efficiency are effectively improved.

In addition, the present disclosure provides an imaging method which does not form part of the appended claims as such, including: providing multiple separate detectors; arranging the multiple separate detectors in form of multi-layers outside the object to be detected to form multiple detector layers; imaging the object to be detected by using the multiple detector layers.

Rays emitted from different positions in the imaging area reach at least one of the multiple separate detectors through different sets of one or more other detector of the multiple separate detectors, wherein the sets of one or more other detector are different in at least one of thicknesses of detector, materials of detector, and number of detectors.

The present disclosure also provides another imaging method which does not form part of the appended claims as such, including: providing multiple separate detectors; arranging the multiple separate detectors in form of multi-layers outside the object to be detected to form multiple detector layers, wherein two adjacent detector layers are spaced from each other by an interval; imaging the object to be detected by using the multiple detector layers.

The present disclosure also provides another imaging method which also does not form part of the appended claims as such, including: providing a collimator and multiple separate detectors including at least two types of detectors; arranging the multiple separate detectors in form of multi-layers outside the object to be detected to form multiple detector layers; disposing the collimator between the object to be detected and a detector layer located innermost along movement direction of the photons; imaging the object to be detected by using the multiple detector layers and the collimator.

The details of the detector, collimator, interval, etc. in the imaging method of the present disclosure are the same as those in the foregoing imaging device embodiment, and will not be repeated here.

## Claims

1. A gamma ray imaging device, comprising a plurality of separate detectors of same type, wherein the plurality of separate detectors form a plurality of detector layers arranged outside an object to be detected (O), rays emitted from different positions (5, 6) in an imaging area reach at least one of the plurality of separate detectors through different sets of one or more other detector of the plurality of separate detectors, the sets of one or more other detector are different in a number of detectors; wherein the plurality of separate detectors are arranged in an array along movement direction of gamma rays and a direction perpendicular to the movement direction of the gamma rays; **characterized in that** two adjacent detector layers are spaced from each other by an interval; and two adjacent detectors in the same layer are spaced from each other by an interval, such that the gamma ray from a position (5, 6) of the object to be detected (O) is allowed to pass through air in the interval between the two adjacent detector layers and the interval between the two adjacent detectors in the same layer to reach the at least one of the plurality of separate detectors.

2. The imaging device according to claim 1, wherein the rays incident from different directions received by the at least one of the plurality of separate detectors have different attenuation ratios due to passing through the sets of one or more other detector which are different in at least one of thickness of detector and number of detectors, so as to determine directions of the rays according to the attenuation ratios of the rays.

3. The imaging device according to claim 1, wherein thicknesses of two adjacent detectors of the plurality of separate detectors are different.

4. The imaging device according to claim 1, wherein the plurality of separate detectors comprise at least two detectors arranged along movement direction of the gamma rays, and the at least two detectors comprise a front detector and a rear detector along the movement direction of the gamma rays, and the front detector is configured to block and collimate the gamma rays moving to the rear detector.

5. The imaging device according to claim 1, wherein distance between any two of the plurality of detectors is configured to cause the gamma rays incident on a detector from different directions have different attenuation ratios.

6. The imaging device according to claim 1, wherein a plurality of detector layers are respectively a first detector layer to an N^{th} detector layer, N≥2, the first detector layer to the N^{th} detector layer are arranged along movement direction of the gamma rays, and the imaging device further comprises an absorption collimator (7) between the first detector layer and an object to be detected (O).

7. An imaging method performed by a gamma ray imaging device, the gamma ray image device comprising:
a plurality of separate detectors of same type, wherein the plurality of separate detectors form a plurality of detector layers arranged outside an object to be detected (O),
wherein rays emitted from different positions (5, 6) in an imaging area reach at least one of the plurality of separate detectors through different sets of one or more other detector of the plurality of separate detectors, the sets of one or more other detector are different in a number of detectors;
wherein the plurality of separate detectors are arranged in an array along movement direction of gamma rays and a direction perpendicular to the movement direction of the gamma rays, wherein two adjacent detector layers are spaced from each other by an interval; and wherein two adjacent detectors in the same layer are spaced from each other by an interval, such that the gamma ray from a position (5, 6) of the object to be detected (O) is allowed to pass through air in the interval between the two adjacent detector layers and the interval between the two adjacent detectors in the same layer to reach the at least one of the plurality of separate detectors;
the method comprising:
providing the plurality of separate detectors,
arranging the plurality of separate detectors in form of multi-layers outside an object to be detected (O) to form the plurality of detector layers;
imaging the object to be detected (O) by using the plurality of detector layers.

## Patentansprüche

1. Vorrichtung zur Bildgebung mittels Gammastrahlen, umfassend eine Vielzahl von getrennten Detektoren gleicher Art, wobei die Vielzahl von getrennten Detektoren eine Vielzahl von Detektorschichten ausbildet, die außerhalb eines zu detektierenden Objekts (O) angeordnet ist, Strahlen, die von unterschiedlichen Positionen (5, 6) in einem Bildgebungsbereich abgegeben werden, mindestens einen der Vielzahl von getrennten Detektoren über unterschiedliche Sätze aus einem oder mehreren anderen Detektoren der Vielzahl von getrennten Detektoren erreichen, sich die Sätze aus einem oder mehreren anderen Detektoren in einer Anzahl von Detektoren unterscheiden; wobei die Vielzahl von getrennten Detektoren in einer Anordnung entlang einer Bewegungsrichtung von Gammastrahlen und einer Richtung senkrecht zu der Bewegungsrichtung der Gammastrahlen angeordnet ist; **dadurch gekennzeichnet, dass** zwei benachbarte Detektorschichten in einem Intervall voneinander beabstandet sind; und zwei benachbarte Detektoren in derselben Schicht in einem Intervall voneinander beabstandet sind, sodass es dem Gammastrahl von einer Position (5, 6) des zu detektierenden Objekts (O) ermöglicht wird, Luft in dem Intervall zwischen den zwei benachbarten Detektorschichten und dem Intervall zwischen den zwei benachbarten Detektoren in derselben Schicht zu passieren, um den mindestens einen der Vielzahl von getrennten Detektoren zu erreichen.

2. Bildgebungsvorrichtung nach Anspruch 1, wobei die Strahlen, die aus unterschiedlichen Richtungen einfallen und von dem mindestens einen der Vielzahl von getrennten Detektoren empfangen werden, unterschiedliche Dämpfungsverhältnisse aufweisen, da sie die Sätze aus einem oder mehreren anderen Detektoren passieren, die sich in mindestens einem von einer Detektordicke und einer Anzahl von Detektoren unterscheiden, um Richtungen der Strahlen gemäß den Dämpfungsverhältnissen der Strahlen zu bestimmen.

3. Bildgebungsvorrichtung nach Anspruch 1, wobei sich Dicken von zwei benachbarten Detektoren der Vielzahl von getrennten Detektoren unterscheiden.

4. Bildgebungsvorrichtung nach Anspruch 1, wobei die Vielzahl von getrennten Detektoren mindestens zwei Detektoren umfasst, die entlang einer Bewegungsrichtung der Gammastrahlen angeordnet sind, und die mindestens zwei Detektoren einen vorderen Detektor und einen hinteren Detektor entlang der Bewegungsrichtung der Gammastrahlen umfassen und der vordere Detektor dazu ausgelegt ist, die Gammastrahlen, die sich zu dem hinteren Detektor bewegen, zu blockieren und zu kollimieren.

5. Bildgebungsvorrichtung nach Anspruch 1, wobei ein Abstand zwischen beliebigen zwei der Vielzahl von Detektoren so ausgelegt ist, dass er bewirkt, dass die Gammastrahlen, die aus unterschiedlichen Richtungen auf einen Detektor einfallen, unterschiedliche Dämpfungsverhältnisse aufweisen.

6. Bildgebungsvorrichtung nach Anspruch 1, wobei eine Vielzahl von Detektorschichten jeweils eine erste Detektorschicht bis eine N-te Detektorschicht ist, N≥2, die erste Detektorschicht bis die N-te Detektorschicht entlang einer Bewegungsrichtung der Gammastrahlen angeordnet sind und die Bildgebungsvorrichtung ferner einen Absorptionskollimator (7) zwischen der ersten Detektorschicht und einem zu detektierenden Objekt (O) umfasst.

7. Bildgebungsverfahren, das durch eine Vorrichtung zur Bildgebung mittels Gammastrahlen durchgeführt wird, wobei die Vorrichtung zur Bildgebung mittels Gammastrahlen Folgendes umfasst:
eine Vielzahl von getrennten Detektoren gleicher Art, wobei die Vielzahl von getrennten Detektoren eine Vielzahl von Detektorschichten ausbildet, die außerhalb eines zu detektierenden Objekts (O) angeordnet ist,
wobei Strahlen, die von unterschiedlichen Positionen (5, 6) in einem Bildgebungsbereich abgegeben werden, mindestens einen der Vielzahl von getrennten Detektoren über unterschiedliche Sätze aus einem oder mehreren anderen Detektoren der Vielzahl von getrennten Detektoren erreichen, wobei sich die Sätze aus einem oder mehreren anderen Detektoren in einer Anzahl von Detektoren unterscheiden;
wobei die Vielzahl von getrennten Detektoren in einer Anordnung entlang einer Bewegungsrichtung von Gammastrahlen und einer Richtung senkrecht zu der Bewegungsrichtung der Gammastrahlen angeordnet ist, wobei zwei benachbarte Detektorschichten in einem Intervall voneinander beabstandet sind; und wobei zwei benachbarte Detektoren in derselben Schicht in einem Intervall voneinander beabstandet sind, sodass es dem Gammastrahl von einer Position (5, 6) des zu detektierenden Objekts (O) ermöglicht wird, Luft in dem Intervall zwischen den zwei benachbarten Detektorschichten und dem Intervall zwischen den zwei benachbarten Detektoren in derselben Schicht zu passieren, um den mindestens einen der Vielzahl von getrennten Detektoren zu erreichen;
wobei das Verfahren Folgendes umfasst:
Bereitstellen der Vielzahl von getrennten Detektoren,
Anordnen der Vielzahl von getrennten Detektoren in Form von mehreren Schichten außerhalb eines zu detektierenden Objekts (O), um die Vielzahl von Detektorschichten auszubilden;
Abbilden des zu detektierenden Objekts (O) unter Verwendung der Vielzahl von Detektorschichten.

## Revendications

1. Dispositif d'imagerie par rayons gamma, comprenant une pluralité de détecteurs distincts de même type, dans lequel la pluralité de détecteurs distincts forme une pluralité de couches de détecteurs disposées à l'extérieur d'un objet à détecter (O), les rayons émis de différentes positions (5, 6) dans une zone d'imagerie atteignent au moins l'un parmi la pluralité de détecteurs distincts à travers différents ensembles d'un ou de plusieurs autres détecteurs de la pluralité de détecteurs distincts, les ensembles d'un ou de plusieurs autres détecteurs étant différents dans un nombre de détecteurs ; dans lequel la pluralité de détecteurs distincts est disposée dans une matrice selon une direction de déplacement des rayons gamma et une direction perpendiculaire à la direction de déplacement des rayons gamma ; **caractérisé en ce que** deux couches de détecteurs adjacentes sont espacées l'une de l'autre d'un intervalle ; et deux détecteurs adjacents dans la même couche sont espacés l'un de l'autre d'un intervalle, de sorte que le rayon gamma en provenance d'une position (5, 6) de l'objet à détecter (O) puisse traverser l'air dans l'intervalle entre les deux couches de détecteurs adjacentes et l'intervalle entre les deux détecteurs adjacents d'une même couche pour atteindre ledit au moins un de la pluralité de détecteurs distincts.

2. Dispositif d'imagerie selon la revendication 1, dans lequel les rayons incidents de directions différentes reçus par ledit au moins un de la pluralité de détecteurs distincts présentent des taux d'atténuation différents dus au passage à travers les ensembles d'un ou de plusieurs autres détecteurs qui diffèrent par au moins l'un des éléments parmi l'épaisseur de détecteur et le nombre de détecteurs, de manière à déterminer des directions des rayons selon les taux d'atténuation des rayons.

3. Dispositif d'imagerie selon la revendication 1, dans lequel les épaisseurs de deux détecteurs adjacents de la pluralité de détecteurs distincts sont différentes.

4. Dispositif d'imagerie selon la revendication 1, dans lequel la pluralité de détecteurs distincts comprend au moins deux détecteurs disposés selon la direction de déplacement des rayons gamma, et lesdits au moins deux détecteurs comprennent un détecteur avant et un détecteur arrière selon la direction de déplacement des rayons gamma, et le détecteur avant est configuré de manière à bloquer et collimater les rayons gamma se déplaçant vers le détecteur arrière.

5. Dispositif d'imagerie selon la revendication 1, dans lequel la distance entre deux quelconques de la pluralité de détecteurs est configurée de manière à faire en sorte que les rayons gamma incidents sur un détecteur en provenance de directions différentes présentent des taux d'atténuation différents.

6. Dispositif d'imagerie selon la revendication 1, dans lequel une pluralité de couches de détecteurs vont respectivement d'une première couche de détecteur à une N^{ième} couche de détecteur, N≥2, la première couche de détecteur à la N^{ième} couche de détecteur sont disposées selon la direction de déplacement des rayons gamma, et le dispositif d'imagerie comprend en outre un collimateur d'absorption (7) entre la première couche de détecteur et un objet à détecter (O).

7. Procédé d'imagerie réalisé par un dispositif d'imagerie par rayons gamma, le dispositif d'imagerie par rayons gamma comprenant :
une pluralité de détecteurs distincts de même type, la pluralité de détecteurs distincts formant une pluralité de couches de détecteurs disposées à l'extérieur d'un objet à détecter (O),
dans lequel des rayons émis de différentes positions (5, 6) dans une zone d'imagerie atteignent au moins l'un parmi la pluralité de détecteurs distincts à travers différents ensembles d'un ou de plusieurs autres détecteurs de la pluralité de détecteurs distincts, les ensembles d'un ou de plusieurs autres détecteurs étant différents dans un nombre de détecteurs ;
dans lequel la pluralité de détecteurs distincts est disposée dans une matrice selon une direction de déplacement des rayons gamma et une direction perpendiculaire à la direction de déplacement des rayons gamma, dans lequel deux couches de détecteurs adjacentes sont espacées l'une de l'autre d'un intervalle, et dans lequel deux détecteurs adjacents d'une même couche sont espacés l'un de l'autre d'un intervalle, de sorte que le rayon gamma en provenance d'une position (5, 6) de l'objet à détecter (O) puisse traverser l'air dans l'intervalle entre les deux couches de détecteurs adjacentes et l'intervalle entre les deux détecteurs adjacents d'une même couche pour atteindre ledit au moins un de la pluralité de détecteurs distincts ;
le procédé comprenant :
la fourniture de la pluralité de détecteurs distincts,
la disposition de la pluralité de détecteurs distincts sous forme de multicouches à l'extérieur d'un objet à détecter (O) pour former la pluralité de couches de détecteurs ;
l'imagerie de l'objet à détecter (O) en utilisant la pluralité de couches de détecteurs.
